# EUROPEAN PATENT APPLICATION

(11) **EP 4 233 782 A1**
(43) Date of publication of application: **30.08.2023**
(21) Application number: 21883038.8
(22) Date of filing: 28.09.2021
(51) Int. Cl.: A61C 9/00, A61C 7/00, A61C 13/00, A61B 5/00

(54) **ORAL IMAGE PROCESSING DEVICE AND ORAL IMAGE PROCESSING METHOD**

(30) Priority: 23.10.2020 KR 20200138596
(71) Applicant: Medit Corp., Seoul 07207 (KR)
(72) Inventor: KIM, Du Su, Seoul 07207 (KR)
(74) Representative: Taor, Simon Edward William
(86) International application number: PCT/KR2021/013237
(87) International publication number: WO 2022/085966

(57) **Abstract**

An intraoral image processing device and an intraoral image processing method are provided. The intraoral image processing method includes obtaining an intraoral image generated by scanning teeth, aligning a teeth model template including a plurality of template teeth to teeth included in the intraoral image, obtaining a plurality of tooth pieces by separating the teeth of the intraoral image, and individualizing the teeth of the intraoral image by collecting one or more tooth pieces corresponding to each of the aligned template teeth.

## Description

### Technical Field

An embodiment of the disclosure relates to an intraoral image processing device and an intraoral image processing method.

Particularly, the embodiment of the disclosure relates to an intraoral image processing device and an intraoral image processing method for individualizing one or more teeth included in an intraoral image.

### Background Art

For dental treatment of a patient, an intraoral scanner may be inserted into an intraoral cavity of the patient, and an intraoral image of the patient may be obtained through the intraoral scanner. The intraoral image obtained through the intraoral scanner may include one or more teeth. For prosthetic treatment or orthodontic treatment, which is a type of dental treatment, it is necessary to perform manipulation, for example, moving each of the teeth included in the intraoral image. Thus, it is necessary to individualize the teeth, for example, by identifying position information of each of the teeth included in the intraoral image and assigning a number to each of the teeth.

### Disclosure

### TECHNICAL PROBLEM

The objective of an embodiment of the disclosure is to provide an intraoral image processing method for individualizing one or more teeth included in an intraoral image, and a device for performing an operation according to the method.

### Technical Solution

An intraoral image processing method according to an embodiment includes obtaining an intraoral image generated by scanning teeth, aligning a teeth model template including a plurality of template teeth to teeth included in the intraoral image, obtaining a plurality of tooth pieces by separating the teeth of the intraoral image, and individualizing the teeth of the intraoral image by collecting one or more tooth pieces corresponding to each of the aligned template teeth.

The individualizing of the teeth of the intraoral image may include obtaining a mapping relationship between each tooth piece of the intraoral image and each of the template teeth by identifying the tooth piece corresponding to each aligned template tooth and collecting the one or more tooth pieces mapped to each template tooth by using the mapping relationship.

The aligning of the teeth model template including the plurality of template teeth to the teeth included in the intraoral image may include positioning the plurality of template teeth of the teeth model template to correspond to the teeth included in the intraoral image.

The obtaining of the plurality of tooth pieces by separating the teeth of the intraoral image may include separating each tooth into one or more tooth pieces by separating the teeth according to a curvature distribution.

The individualizing of the teeth of the intraoral image may include identifying each tooth piece corresponding to each template tooth by using an orientation condition of each template tooth and each tooth piece.

The identifying of each tooth piece corresponding to each template tooth by using the orientation condition may include identifying a crossing point at which a normal vector on each vertex of a three-dimensional (3D) mesh forming the template tooth intersects a 3D mesh forming the tooth piece, and based on an angle between a normal vector on the identified crossing point with respect to the tooth piece and the normal vector on the vertex being less than a threshold value, determining that the tooth piece corresponds to the template tooth.

The individualizing of the teeth of the intraoral image may include identifying each tooth piece corresponding to each template tooth by using a distance condition of each template tooth and each tooth piece, and the identifying of each tooth piece corresponding to each template tooth may include identifying a crossing point at which a normal vector on each vertex of a 3D mesh forming the template tooth intersects a 3D mesh forming the tooth piece, and based on a distance from the vertex to the crossing point being less than a threshold value, determining that the tooth piece corresponds to the template tooth.

The individualizing of the teeth of the intraoral image may include identifying each tooth piece corresponding to each template tooth by using a distance condition and an orientation condition of each template tooth and each tooth piece, and the identifying of each tooth piece corresponding to each template tooth by using the distance condition and the orientation condition may include identifying, as a crossing point at which a normal vector on each vertex of a 3D mesh forming the template tooth intersects a 3D mesh forming the tooth piece, the crossing point within a predetermined distance from the vertex, and based on an angle between a normal vector on the identified crossing point with respect to the tooth piece and the normal vector on the vertex being less than a threshold value, determining that the tooth piece corresponds to the template tooth.

An intraoral image processing device according to an embodiment includes a processor and a memory, wherein the processor is configured to execute one or more instructions stored in the memory to obtain an intraoral image generated by scanning teeth, align a teeth model template including a plurality of template teeth to teeth included in the intraoral image, obtain a plurality of tooth pieces by separating the teeth of the intraoral image, and individualize the teeth of the intraoral image by collecting one or more tooth pieces corresponding to each of the aligned template teeth.

A computer-readable recording medium according to an embodiment has recorded thereon a program including one or more instructions for executing, on a computer, an intraoral image processing method including obtaining an intraoral image generated by scanning teeth, aligning a teeth model template including a plurality of template teeth to teeth included in the intraoral image, obtaining a plurality of tooth pieces by separating the teeth of the intraoral image, and individualizing the teeth of the intraoral image by collecting one or more tooth pieces corresponding to each of the aligned template teeth.

### Advantageous Effects

An intraoral image processing method and a device for performing operations according to the intraoral image processing method, according to an embodiment of the disclosure, may relatively more accurately and conveniently individualize teeth by providing a method of individualizing teeth included in an intraoral image by using a teeth model template.

### Description of Drawings

The disclosure may be easily understood based on the combination of the following detailed descriptions and the accompanying drawings, and reference numerals indicate structural elements.
FIG. 1 is a diagram for describing an intraoral image processing system, according to an embodiment of the disclosure.
FIG. 2 is a reference diagram for describing a method of individualizing teeth included in an intraoral image by using a template, according to an embodiment.
FIG. 3 is a block diagram of a data processing device according to an embodiment.
FIG. 4 is a flowchart of an intraoral image processing method of a data processing device, according to an embodiment.
FIG. 5 illustrates an example of a three-dimensional (3D) mesh structure of resultant data obtained by using a 3D scanner, according to an embodiment.
FIG. 6 is a reference diagram for describing a method of separating an intraoral image into a tooth mass and gingiva, according to an embodiment.
FIG. 7 is a reference diagram for describing a method of separating a tooth mass into a plurality of tooth pieces, according to an embodiment.
FIG. 8 is a map illustrating a curvature distribution corresponding to an intraoral image, according to an embodiment.
FIG. 9 is a reference diagram for describing a method of aligning a teeth model template to a tooth mass of an intraoral image, according to an embodiment.
FIG. 10 is a reference diagram for describing a method of obtaining a mapping relationship between each tooth piece of a tooth mass and each of template teeth, according to an embodiment.
FIG. 11 illustrates a portion of data in which a tooth mass and a teeth model template are aligned to each other, according to an embodiment.
FIG. 12 is a reference diagram for describing a method of obtaining a mapping relationship between each tooth piece of a tooth mass and each template tooth by using an orientation condition and a distance condition, according to an embodiment.
FIG. 13 is a reference diagram for describing a method of obtaining a mapping relationship between each tooth piece of a tooth mass and each template tooth by using an orientation condition and a distance condition, according to an embodiment.
FIG. 14 is a reference diagram for describing a case where one tooth piece is repeatedly mapped to a plurality of template teeth, according to an embodiment.
FIG. 15 is a reference diagram for describing a method of individualizing teeth of an intraoral image by collecting one or more tooth pieces mapped to template teeth by using a mapping relationship between the tooth pieces and the template teeth, according to an embodiment.

### Mode for Invention

In this specification, principles of the disclosure are described and embodiments are disclosed, in order to clarify the scope of the claims of the disclosure and clearly convey the disclosure for one of ordinary skill in the art to implement the disclosure. Embodiments of the disclosure may be implemented in various forms.

Throughout the specification, like reference numerals refer to like elements. Not all elements of the embodiments are described in this specification, and general aspects in the art or the same aspects of the embodiments are not described. The term "part" or "portion" used in the specification may be implemented as software or hardware, and according to embodiments, a plurality of "units" may be implemented as one unit (element), or one "unit" may include a plurality of units (elements). Hereinafter, by referring to the accompanying drawings, the operating principles and the embodiments of the disclosure are described.

In this specification, an image may include an image indicating at least one tooth or an intraoral cavity including at least one tooth and an image (hereinafter, "an intraoral image") indicating a teeth model.

Also, in this specification, an image may include a two-dimensional (2D) image with respect to an object or a three-dimensional (3D) model or a 3D image three-dimensionally representing an object. Also, in this specification, an image may denote data needed to two-dimensionally or three-dimensionally represent an object, for example, raw data, etc. obtained from at least one image sensor. In detail, the raw data is 2D or 3D data obtained to generate an intraoral image. When an object, which is an intraoral cavity of a patient, is scanned by using a 3D scanner, the raw data may be data (for example, 2D data) obtained by at least one image sensor included in the intraoral scanner. The 3D scanner may include the intraoral scanner, a table scanner, a computerized tomography (CT) scanner, etc.

Hereinafter, the intraoral scanner will be described as an example.

In this specification, an "object" may include a tooth, gingiva, at least a portion of an intraoral cavity, and/or an artificial structure (for example, an orthodontic appliance, an implant, an artificial tooth, an orthodontic auxiliary instrument inserted into the intraoral cavity, etc.) which may be inserted into the intraoral cavity. Here, the orthodontic appliance may include at least one of a bracket, an attachment, an orthodontic screw, an orthodontic appliance for tongue, and a removable orthodontic-maintenance appliance.

Hereinafter, embodiments are described in detail with reference to the drawings.

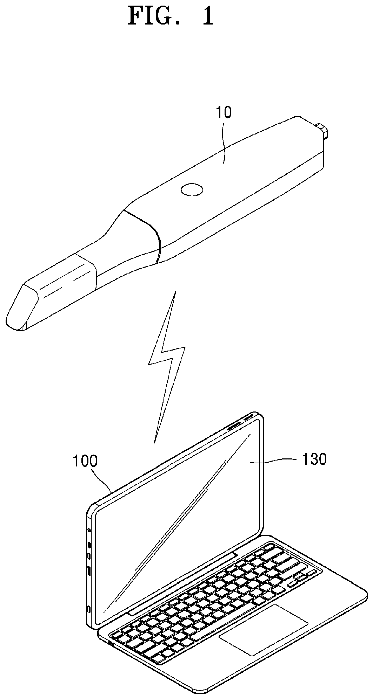

FIG. 1 is a diagram for describing an intraoral image processing system, according to an embodiment.

Referring to FIG. 1, an intraoral scanner 10 is a medical appliance for obtaining an image with respect to an intraoral cavity.

In detail, the intraoral scanner 10 may be a device inserted into an intraoral cavity and scanning teeth in a non-contact way for obtaining an image with respect to an intraoral cavity including one or more teeth. Also, the intraoral scanner 10 may have a form that is possible to be inserted into and withdrawn from an intraoral cavity and may scan an internal state of the intraoral cavity of a patient by using at least one image sensor (for example, an optical camera, etc.). The intraoral scanner 10 may obtain surface information with respect to an object, as raw data, in order to image a surface of at least one of objects including a tooth in the intraoral cavity, a gum, and an artificial structure (e.g., an orthodontic device including a bracket, a wire, etc., an implant, an artificial tooth, an orthodontic auxiliary instrument inserted into the intraoral cavity, etc.) which may be inserted into the intraoral cavity. Also, the intraoral scanner 10 may obtain an intraoral image not only by directly scanning the intraoral cavity of a patient, but also by scanning a teeth model or a plastic model obtained by impression-taking on the patient.

Image data obtained by the intraoral scanner 10 may be transmitted to a data processing device 100 connected to the intraoral scanner 10 through a wired or wireless communication network.

The data processing device 100 may include all types of electronic devices which are connected to the intraoral scanner 10 through a wired or wireless communication network and capable of receiving, from the intraoral scanner 10, a 2D image obtained by scanning an intraoral cavity, and generating, processing, displaying, and/or transmitting an intraoral image based on the received 2D image. In addition to receiving the intraoral image obtained by the intraoral scanner 10, the data processing device 100 may receive, from a 3D model scanner, a teeth model image obtained by the 3D model scanner by scanning a teeth model.

Based on the 2D image data received from the intraoral scanner 10, the data processing device 100 may generate at least one of information generated by processing the 2D image data and an intraoral image generated by processing the 2D image data and may display the generated information or intraoral image through a display 130.

The data processing device 100 is illustrated as a laptop computer in FIG. 1. However, the data processing device 100 is not limited thereto. The data processing device 100 may include, but is not limited to, a computing device, such as a smartphone, a desktop computer, a personal digital assistant (PDA), a tablet personal computer (PC), etc.

Also, the data processing device 100 may also be implemented as a server (or a server device) configured to process an intraoral image.

Also, the intraoral scanner 10 may directly transmit the raw data obtained through intraoral scanning to the data processing device 100. In this case, the data processing device 100 may generate, based on the received raw data, a 3D intraoral image three-dimensionally representing an intraoral cavity. Also, the "3D intraoral image" may be generated by three-dimensionally modeling an internal structure of the intraoral cavity based on the received raw data, and thus, may also be referred to as a "3D intraoral model" or a "3D intraoral image." Hereinafter, models or images two-dimensionally and three-dimensionally indicating an intraoral cavity are commonly referred to as "intraoral images."

In addition, the data processing device 100 may analyze, process, display, and/or transmit, to an external device, the generated intraoral image.

As another example, the intraoral scanner 10 may obtain the raw data through intraoral scanning, may generate an image corresponding to the intraoral cavity, which is the object, by processing the obtained raw data, and may transmit the generated image to the data processing device 100. In this case, the data processing device 100 may analyze, process, display, and/or transmit the received image.

According to an embodiment of the disclosure, detailed descriptions are given below based on the data processing device 100 being an electronic device capable of generating and displaying an intraoral image three-dimensionally indicating an intraoral cavity including one or more teeth.

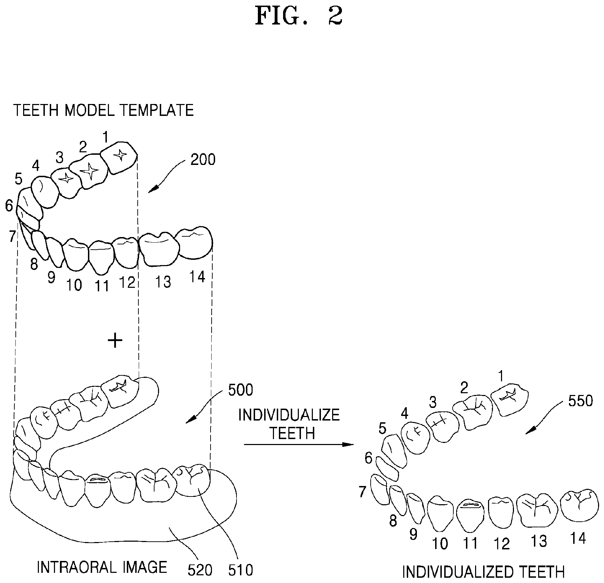

FIG. 2 is a reference diagram for describing a method of individualizing teeth included in an intraoral image by using a teeth model template, according to an embodiment.

An intraoral image 500 may include an intraoral image obtained by using the intraoral scanner 10 with respect to an intraoral cavity of a patient, an intraoral image obtained by scanning a teeth model of a patient by using a 3D model scanner, or the like, etc.

The intraoral image 500 may image the entire surface in the intraoral cavity of the patient, and thus, one or more teeth, gingiva 520 surrounding the teeth, etc. are altogether imaged.

The intraoral scanner 10 may obtain 2D data indicating surface information with respect to the intraoral cavity, which is an object. Also, the data processing device 100 may generate the intraoral image 500 based on the 2D data obtained by the intraoral scanner 10.

The intraoral image 500 may have a polygonal mesh structure in which adjacent vertexes are polygonised in a point cloud having, as the vertexes, coordinates of points obtained by the intraoral scanner 10 by scanning the object. The polygons of the mesh structure may include a triangle, a quadrangle, a pentagon, etc. According to an embodiment, the polygon may be a triangle. In the intraoral image 500 having the polygonal mesh structure generated by the data processing device 100 as described above, the teeth and the gingiva 520 may not be separated from each other, and the teeth may be recognized as a tooth mass 510 in which each of the plurality of teeth is not separated. Alternatively, according to another embodiment, the data processing device 100 may separate, by using artificial intelligence (Al), a teeth area and gingiva area in a 2D image obtained through a scanning process, so that the intraoral image 500 generated by the data processing device 100 may automatically have a state in which the tooth mass and the gingiva are separated from each other.

A teeth model template 200 indicates template model data in which teeth have ideal shapes and are arranged at ideal positions, and numbers are respectively assigned to the teeth. For example, each template tooth of the teeth model template 200 is sequentially assigned with one of tooth numbers 1, 2, ..., and 14, starting from the left.

The data processing device 100 may perform data-processing on the teeth of the intraoral image 500 by using the teeth model template 200 to individualize the teeth of the intraoral image and obtain individualized teeth 550. To individualize the teeth of the intraoral image may denote to separate the teeth from the gingiva in the intraoral image and may also denote to obtain information about each of the teeth. The information about each tooth may include information about a shape of each tooth, information about a position of each tooth, and information about a number of each tooth. The individualization of the teeth of the intraoral image may also be referred to as segmentation or classification of the teeth of the intraoral image. As described above, because the teeth of the intraoral image are individualized, the data processing device 100 may perform a procession operation, such as deleting each tooth, moving each tooth, or adding an additional tooth, by using the individualized teeth 550.

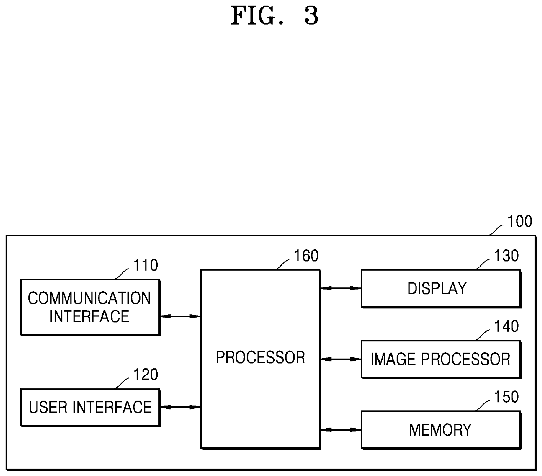

FIG. 3 is a block diagram of the data processing device 100 according to an embodiment.

Referring to FIG. 3, the data processing device 100 may include a communication interface 110, a user interface 120, a display 130, an image processor 140, a memory 150, and a processor 160.

The communication interface 110 may perform communication with at least one external electronic device through a wired or wireless communication network. In detail, the communication interface 110 may perform communication with the intraoral scanner 10 according to control by the processor 160. The communication interface 110 may perform communication with an external electronic device or a server connected to the communication interface 110 through a wired or wireless communication network, according to control by the processor 160.

The communication interface 110 may communicate with the external electronic device (for example, an intraoral scanner, a server, or an external medical device) through the wired or wireless communication network. In detail, the communication interface 110 may include at least one short-range wireless communication module performing communication according to the communication standards, such as Bluetooth, Wifi, Bluetooth low energy (BLE), near-field communication (NFC)/radio-frequency identification (RFID), Wifi-direct, ultra-wide band (UWB), or Zigbee.

Also, the communication interface 110 may further include a remote communication module performing communication with a server for supporting remote communication according to the remote communication standards. In detail, the communication interface 110 may include the remote communication module performing communication through a network for Internet communication. Also, the communication interface 110 may include a remote communication module performing communication through a communication network according to the communication standards, such as the 3^{rd} generation (3G), the 4^{th} generation (4G), and/or the 5^{th} generation (5G).

Also, in order to communicate with the external electronic device (for example, the intraoral scanner, etc.) in a wired manner, the communication interface 110 may include at least one port to be connected to the external electronic device through a wired cable. Accordingly, the communication interface 110 may perform communication with the external electronic device connected in a wired manner thorough the at least one port.

The user interface 120 may receive a user input for controlling the data processing device 100. The user interface 120 may include a user input device including a touch panel configured to sense a touch of a user, a button configured to receive a push manipulation of the user, a mouse or a keyboard configured to indicate or select a point on a user interface screen, or the like, but is not limited thereto.

Also, the user interface 120 may include a voice recognition device for voice recognition. For example, the voice recognition device may include a microphone, and the voice recognition device may receive a user's voice command or voice request. Thus, the processor 160 may perform a control operation such that an operation corresponding to the voice command or the voice request may be performed.

The display 130 may display a screen. In detail, the display 130 may display a predetermined screen according to control by the processor 160. In detail, the display 130 may display a user interface screen including an intraoral image generated based on data obtained by the intraoral scanner 10 by scanning an intraoral cavity of a patient. Alternatively, the display 130 may display a user interface screen including information related to dental treatment of the patient.

The image processor 140 may perform operations for generating and/or processing an image. In detail, the image processor 140 may receive the raw data obtained by the intraoral scanner 10 and may generate the intraoral image based on the received data. In detail, the image processor 140 may obtain individualized teeth by individualizing teeth included in the intraoral image of the patient by using a teeth model template. The image processor 140 may be provided separately from the processor 160 as illustrated in FIG. 3, or the image processor 140 may be included in the processor 160.

The memory 150 may store at least one instruction. Also, the memory 150 may store at least one instruction executed by a processor. Also, the memory 150 may store at least one program executed by the processor 160. Also, the memory 150 may store data (for example, the raw data, etc. obtained through intraoral scanning) received from the intraoral scanner. Alternatively, the memory may store the intraoral image three-dimensionally indicating the intraoral cavity. According to an embodiment, the memory 150 may include one or more instructions for obtaining the individualized teeth by individualizing the teeth included in the intraoral image by using the teeth model template. According to an embodiment, the memory 150 may include one or more instructions for performing the method of obtaining the individualized teeth by individualizing the teeth included in the intraoral image by using the teeth model template, the method being disclosed in this specification.

The processor 160 may execute the at least one instruction stored in the memory 150 to control intended operations to be performed. Here, the at least one instruction may be stored in an internal memory of the processor 160 or in the memory 150 included in the data processing device 100 separately from the processor 160.

In detail, the processor 160 may execute the at least one instruction to control one or more components included in the data processing device 100 to perform intended operations. Thus, although the processor 160 is described as performing predetermined operations, it may denote that the processor 160 controls the one or more components included in the data processing device 100 to perform predetermined operations.

According to an embodiment, the processor 160 may execute the one or more instructions stored in the memory 150 to: obtain an intraoral image generated by scanning teeth; align a teeth model template including a plurality of template teeth to the teeth included in the intraoral image; obtain a plurality of tooth pieces by separating the teeth of the intraoral image according to a curvature distribution; and individualize the teeth of the intraoral image by collecting one or more tooth pieces corresponding to each of aligned template teeth.

According to an embodiment, the processor 160 may execute the one or more instructions stored in the memory 150 to: obtain a mapping relationship between each tooth piece of the intraoral image and each template tooth by identifying the tooth piece corresponding to each aligned template tooth; and collect the one or more tooth pieces mapped to each template tooth by using the mapping relationship.

According to an embodiment, the processor 160 may execute the one or more instructions stored in the memory 150 to: separate the teeth from gingiva in the intraoral image; and separate each tooth into one or more tooth pieces by separating the teeth according to a curvature distribution.

According to an embodiment, the processor 160 may execute the one or more instructions stored in the memory 150 to: position the plurality of template teeth of the teeth model template to correspond to the teeth included in the intraoral image.

According to an embodiment, the processor 160 may execute the one or more instructions stored in the memory 150 to: identify each tooth piece corresponding to each template tooth by using an orientation condition of each template tooth and each tooth piece.

According to an embodiment, the processor 160 may execute the one or more instructions stored in the memory 150 to: identify a crossing point at which a normal vector at each vertex of a 3D mesh forming the template tooth intersects a 3D mesh forming the tooth piece; and based on an angle between the normal vector on the identified crossing point with respect to the tooth piece and the normal vector on the vertex being less than a threshold value, determine that the tooth piece corresponds to the template tooth.

According to an embodiment, the processor 160 may execute the one or more instructions stored in the memory 150 to: identify each tooth piece corresponding to each template tooth by using a distance condition of each template tooth and each tooth piece.

According to an embodiment, the processor 160 may execute the one or more instructions stored in the memory 150 to: with respect to the identifying using the distance condition, identify a crossing point at which a normal vector at each vertex of a 3D mesh forming the template tooth intersects a 3D mesh forming the tooth piece; and based on a distance from the vertex to the crossing point being less than a threshold value, determine that the tooth piece corresponds to the template tooth.

According to an embodiment, the processor 160 may execute the one or more instructions stored in the memory 150 to: identify each tooth piece corresponding to each template tooth by using the orientation condition and the distance condition of each template tooth and each tooth piece.

According to an embodiment, the processor 160 may execute the one or more instructions stored in the memory 150 to: with respect to the identifying of each tooth piece corresponding to each template tooth by using the orientation condition and the distance condition, identify a crossing point within a predetermined distance from the vertex, as the crossing point at which the normal vector at each vertex of the 3D mesh forming the template tooth intersects the 3D mesh forming the tooth piece; and based on an angle between the normal vector on the identified crossing point with respect to the tooth piece and the normal vector on the vertex being less than threshold value, determine that the tooth piece corresponds to the template tooth.

According to an embodiment, the processor 160 may execute the one or more instructions stored in the memory 150 to: when the one or more tooth pieces mapped to each template tooth are collected, ignore a tooth piece repeatedly mapped to the plurality of template teeth.

According to an embodiment, the processor 160 may be implemented as a form of processor internally including at least one internal processor and a memory device (for example, random-access memory (RAM), read-only memory (ROM), etc.) for storing at least one of a program, an instruction, a signal, and data to be processed or used by the internal processor.

Also, the processor 160 may include a graphic processing unit (GPU) for processing graphics data corresponding to video data. Also, the processor 160 may be realized as a system on chip (SoC) combining a core and a GPU. Also, the processor 160 may include a multi-core including more cores than a single-core. For example, the processor 160 may include a dual core, a triple core, a quad core, a hexa core, an octa core, a deca core, a dodeca core, a hexadecimal core, or the like.

According to an embodiment of the disclosure, the processor 160 may generate the intraoral image based on a 2D image received from the intraoral scanner 10.

In detail, according to control by the processor 160, the communication interface 110 may receive the data obtained by the intraoral scanner 10, for example, the raw data obtained through intraoral scanning. Also, the processor 160 may generate a 3D intraoral image three-dimensionally indicating the intraoral cavity, based on the raw data received from the communication interface 110. For example, in order to restore the 3D image according to optical triangulation, the intraoral scanner 10 may include at least one camera, and according to a specific embodiment, may include an L camera corresponding to a left field of view and an R camera corresponding to a right field of view. Also, the intraoral scanner 10 may obtain L image data corresponding to the left field of view and R image data corresponding to the right field of view through the L camera and the R camera, respectively. Further, the intraoral scanner may transmit the raw data including the L image data and the R image data to the communication interface 110 of the data processing device 100.

Thereafter, the communication interface 110 may transmit the received raw data to the processor 160, and the processor 160 may generate the intraoral image three-dimensionally indicating the intraoral cavity, based on the received raw data.

Also, the processor 160 may directly receive the intraoral image three-dimensionally indicating the intraoral cavity from an external server, a medical device, etc. by controlling the communication interface 110. In this case, the processor 160 may not generate the 3D intraoral image based on the raw data and may obtain the 3D intraoral image.

According to an embodiment of the disclosure, that the processor 160 performs "extraction," "obtaining," "generating" operations, etc. may not only denote that the processor 160 directly performs the described operations by executing one or more instructions, but may also denote that the processor 16t0 controls other components to perform the described operations.

In order to realize one or more embodiments disclosed in this specification, the data processing device 100 may include only some of the components illustrated in FIG. 3 or may include more components than the components illustrated in FIG. 3.

Also, the data processing device 100 may store and execute exclusive software synchronized with the intraoral scanner 10. Here, the exclusive software may be referred to as an exclusive program, an exclusive tool, or an exclusive application. When the data processing device 100 operates in synchronization with the intraoral scanner 10, the exclusive software stored in the data processing device 100 may be connected with the intraoral scanner 10 and may receive, in real time, pieces of data obtained through intraoral scanning. For example, there may be exclusive software for processing data obtained through intraoral scanning, in the case of the i500 product, which is an intraoral scanner of Medit. In detail, Medit manufactures and distributes "Medit link," which is the software for processing, managing, using, and/or transmitting data obtained by an intraoral scanner (for example, i500). Here, the "exclusive software" refers to a program, a tool, or an application operable in synchronization with an intraoral scanner, and thus, the "exclusive software" may be shared by various intraoral scanners developed and sold by various manufacturers. Also, the described exclusive software may be separately manufactured and distributed from the intraoral scanner performing intraoral scanning.

The data processing device 100 may store and execute the exclusive software corresponding to the i500 product. The exclusive software may perform one or more operations for obtaining, processing, storing, and/or transmitting the intraoral image. Here, the exclusive software may be stored in the processor. Also, the exclusive software may provide a user interface for using the data obtained by the intraoral scanner. Here, a screen of the user interface provided by the exclusive software may include the intraoral image generated according to an embodiment of the disclosure.

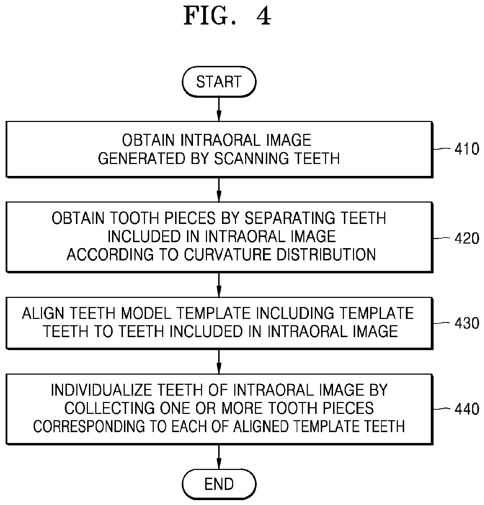

FIG. 4 is a flowchart of an intraoral image processing method of a data processing device, according to an embodiment of the disclosure. The intraoral image processing method illustrated in FIG. 4 may be performed by the data processing device 100. Thus, the intraoral image processing method illustrated in FIG. 4 may be a flowchart of operations of the data processing device 100.

Referring to FIG. 4, in operation 410, the data processing device 100 may obtain an intraoral image generated by scanning teeth.

According to an embodiment, the data processing device 100 may receive 2D data generated by scanning the teeth, from the intraoral scanner 10 as illustrated in FIG. 1, and may generate an intraoral image based on the received 2D data.

According to an embodiment, the data processing device 100 may receive the intraoral image generated based on the 2D data obtained by scanning the teeth from the intraoral scanner 10.

According to an embodiment, the data processing device 100 may obtain an intraoral image stored in the memory.

In operation 420, the data processing device 100 may obtain a plurality of tooth pieces by segmenting the teeth included in the intraoral image according to a curvature distribution.

According to an embodiment, the data processing device 100 may separate the intraoral image 500 into a tooth mass 510 and gingiva 520 according to a curvature distribution. According to another embodiment, the data processing device 100 may automatically separate the intraoral image 500 into the tooth mass 510 and the gingiva 520 by using a neural network using Al.

According to an embodiment, the data processing device 100 may separate the tooth mass 510 into a plurality of tooth pieces according to a curvature distribution. Here, one tooth may also be separated into two or more tooth pieces according to the curvature distribution. Thus, for example, the tooth mass 510 may be separated into 30 to 50 tooth pieces.

In operation 430, the data processing device 100 may align the teeth model template 200 including the plurality of template teeth to the teeth of the intraoral image.

According to an embodiment, when the data processing device 100 aligns the teeth model template 200 to the teeth of the intraoral image, the data processing device 100 may use various automatic aligning algorithms and, for example, may use an iterative closest point (ICP) algorithm.

FIG. 4 illustrates that the tooth pieces are separated in operation 420, and the teeth model template is aligned in operation 430. However, embodiments are not limited thereto. The operation of separating the tooth pieces and the operation of aligning the teeth model template may be performed in a parallel manner, or after the operation of aligning the teeth model template, the operation of separating the tooth pieces may be performed.

In operation 440, the data processing device 100 may individualize the teeth of the intraoral image by collecting one or more tooth pieces corresponding to each aligned template tooth.

According to an embodiment, based on the template teeth aligned to the teeth of the intraoral image, the data processing device 100 may identify a template tooth corresponding to a tooth piece by using an orientation condition (or a normal vector condition) of the template tooth and the tooth piece. The orientation condition (or the normal vector condition) may denote a condition for identifying the tooth piece corresponding to the template tooth by using a normal vector of the template tooth and a normal vector of the tooth piece.

According to an embodiment, the data processing device 100 may identify the tooth piece corresponding to the template tooth by using the normal vector of the template tooth and the normal vector of the tooth piece, the normal vectors being as the orientation condition.

According to an embodiment, the data processing device 100 may identify whether the template tooth and the tooth piece correspond to each other by using an angle between the normal vector at a vertex of each template tooth and the normal vector at a crossing point at which the normal vector meets the tooth piece of the intraoral image.

According to an embodiment, when the angle between the normal vector on the vertex of each template tooth and the normal vector on the crossing point at which the normal vector meets the tooth piece of the intraoral image is less than a threshold angle, the data processing device 100 may identify that the template tooth and the tooth piece correspond to each other, and when the angle is greater than or equal to the threshold angle, the data processing device 100 may identify that the template tooth and the tooth piece do not correspond to each other.

According to an embodiment, the data processing device 100 may identify whether the template tooth and the tooth piece correspond to each other by using a condition of a distance from the vertex of each template tooth to the crossing point at which the normal vector on the vertex meets the tooth piece of the intraoral image.

According to an embodiment, the data processing device 100 may identify a crossing point of the normal vector at each vertex of a 3D mesh forming the template tooth and a 3D mesh forming the tooth piece, and when the distance from the vertex to the crossing point is less than a threshold value, may determine that the tooth piece corresponds to the template tooth.

According to an embodiment, based on the template teeth aligned to the tooth pieces of the intraoral image, the data processing device 100 may identify the template tooth corresponding to the tooth piece by using the distance condition and the orientation condition of the template tooth and the tooth piece.

According to an embodiment, the data processing device 100 may identify, as the tooth piece corresponding to the template tooth, a tooth piece within a predetermined distance from the vertex of each template tooth, the tooth piece satisfying the orientation condition described above.

According to an embodiment, when the plurality of template teeth are mapped to one tooth piece, the data processing device 100 may not use and ignore the tooth piece commonly mapped to the plurality of template teeth, with respect to the collecting of the tooth pieces.

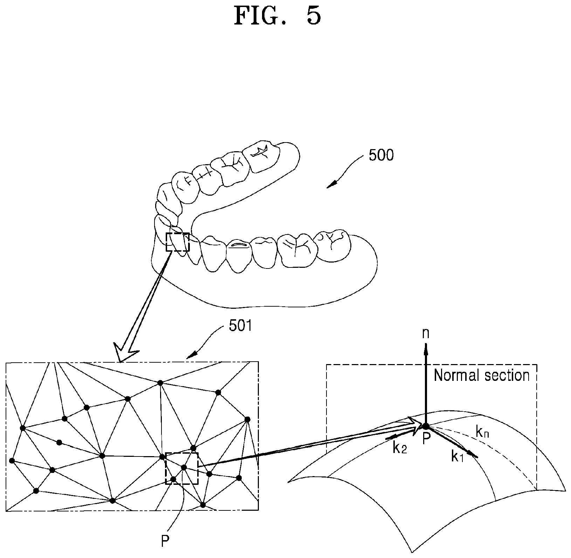

The intraoral image processing method according to an embodiment of the disclosure will be described in detail below with reference to FIGS. 5 to 15.

The data processing device 100 may obtain a plurality of tooth pieces by separating teeth included in an intraoral image according to a curvature distribution. Here, first, the data processing device 100 may separate a tooth mass and gingiva by separating the teeth included in the intraoral image according to the curvature distribution. Also, the data processing device 100 may separate the tooth mass into the plurality of tooth pieces according to the curvature distribution.

First, the concepts of a 3D mesh structure based on resultant data obtained by using an intraoral scanner and the curvature distribution are described.

FIG. 5 illustrates an example of a 3D mesh structure based on resultant data obtained by using an intraoral scanner, according to an embodiment.

When 2D data is obtained by using the intraoral scanner, the data processing device 100 may calculate coordinates of a plurality of examined surface points by using triangulation. As the surfaces of an object are scanned by using the intraoral scanner moving along the surfaces of the object, the amount of scanned data may be increased, and the coordinates of the surfaces points may be accumulated. As a result of obtaining the image, a point cloud of vertexes may be identified to indicate a range of the surfaces. Points on the point cloud may indicate actually measured points on a 3D surface of the object. A surface structure may be approximated as adjacent vertexes on the point cloud form a polygonal mesh connected by a line segment. The polygonal mesh may be variously determined, for example, as a triangular mesh, a quadrangular mesh, a pentagonal mesh, etc. A relationship between a polygon and an adjacent polygon of this mesh model may be used to extract the characteristics of a boundary of a tooth, for example, a curvature, a minimum curvature, an edge, a spatial relationship, etc.

Referring to FIG. 5, a region 501 of the intraoral image 500 may be formed as a triangular mesh generated by connecting a plurality of vertexes and adjacent vertexes forming a point cloud by using lines.

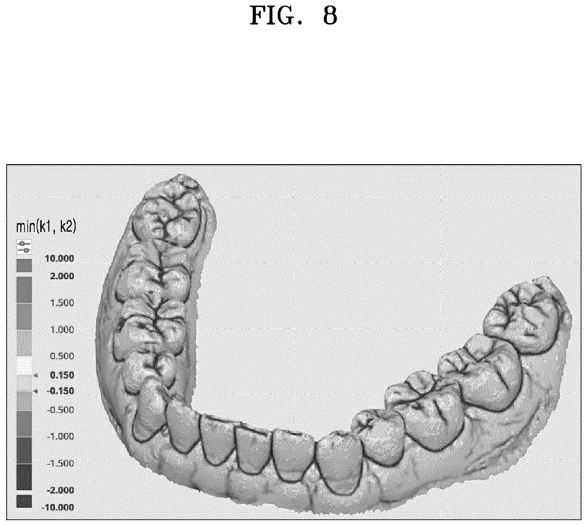

FIGS. 5 and 8 are reference diagrams for describing an operation of separating teeth and gingiva and segmenting a tooth mass by using a curvature distribution. In the diagrams, principal curvatures of a predetermined point P on a surface may indicate a criterion for indicating degrees in which the surface is bent from the predetermined point P in different directions. In detail, a normal vector may be defined on each point P of a 3D surface, and a plane including the normal vector on each point P may be referred to as a normal plane, wherein all of normal planes may have a normal vector which is tangential to a surface on the point P.

When a surface around the point P is cut into different normal planes, a curvature of a curve generated by each cross-section may have a different value from each other, and one or more of these curvatures, which have the unique characteristic, are principal curvatures k1 and k2.

The principal curvatures k1 and k2 on the point P may be defined as below.

k1 indicates a curvature having a greatest absolute value of curvatures of a great number of cross-sections formed by a curve and a normal plane, which is a plane including a normal vector on a predetermined point P on a curved surface.

k2 indicates a curvature assessed on a normal plane orthogonal to the normal plane on which k1 is assessed.

Each principal curvature corresponds to a 1/curvature on each normal plane.

FIG. 8 is a map illustrating a curvature distribution corresponding to an intraoral image, according to an embodiment.

Referring to FIG. 8, the map illustrates a distribution min (k1, k2) of a less value of the principal curvatures k1 and k2, by using the principal curvatures k1 and k2 assessed on each vertex of a mesh structure forming the intraoral image. For example, the data processing device 100 may separate teeth and gingiva and separate a tooth mass into a plurality of tooth pieces by cutting, and segmenting, into pieces, a portion of the map illustrated in FIG. 8, the portion having a value of the distribution min (k1, k2), which is less than 2, according to an embodiment.

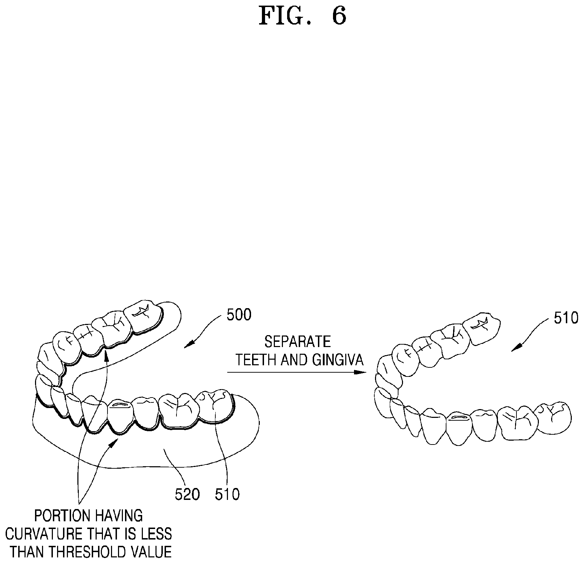

FIG. 6 is a reference diagram for describing a method of separating an intraoral image into a tooth mass and gingiva, according to an embodiment.

Referring to FIG. 6, the intraoral image 500 obtained by an intraoral scanner may include the tooth mass 510 and the gingiva 520, and thus, the data processing device 100 may separate the tooth mass 510 from the gingiva 520 by separating the intraoral image 500 according to a curvature distribution. The data processing device 100 may determine an appropriate curvature threshold for separating a boundary between the tooth mass 510 and the gingiva 520 and may separate a portion having a curvature that is less than the determined curvature threshold to separate the tooth mass 510 and the gingiva 520.

The data processing device 100 may separate the tooth mass and the gingiva from each other based on a predetermined curvature, and then, may perform a subsequent process by using data of a portion corresponding to the tooth mass.

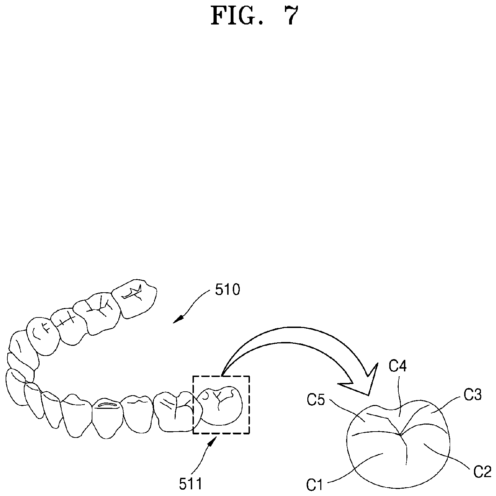

FIG. 7 is a reference diagram for describing a method of separating a tooth mass into a plurality of tooth pieces, according to an embodiment.

Referring to FIG. 7, the tooth mass 510 separated from the gingiva 520 in the intraoral image 500 may be separated into a plurality of tooth pieces according to a curvature. When the tooth mass 510 is separated into the plurality of tooth pieces, not only the teeth included in the tooth mass 510 may be separated into the tooth pieces, but also one tooth may be separated into the plurality of tooth pieces according to the curvature. The data processing device 100 may determine an appropriate curvature threshold for separating a boundary of each tooth piece in the tooth mass 510 and may separate a portion having a curvature that is less than the determined curvature threshold to separate the tooth mass 510 into the plurality of tooth pieces. For example, referring to FIG. 7, a tooth 511 may be separated into five tooth pieces C1, C2, C3, C4, and C5. Thus, the tooth mass 510 may be separated, for example, into 30 to 50 tooth pieces.

According to an embodiment, the data processing device 100 may determine the curvature threshold used for separating the tooth mass and the gingiva from each other in the intraoral image and the curvature threshold used for separating the tooth mass into the plurality of tooth pieces to have the same value as each other and may simultaneously separate the plurality of tooth pieces in the tooth mass 510.

According to another embodiment, the data processing device 100 may determine the curvature threshold used for separating the tooth mass from the gingiva in the intraoral image to have a first value and the curvature threshold used for separating the tooth mass into the plurality of tooth pieces to have a second value. By doing so, the data processing device 100 may first separate the tooth mass and the gingiva from each other in the intraoral image by using the curvature threshold having the first value, and then, may separate the tooth mass into the plurality of tooth pieces by using the curvature threshold having the second value.

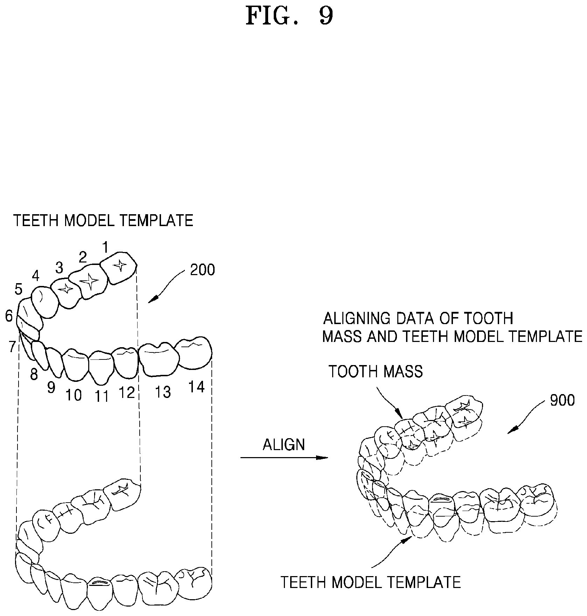

FIG. 9 is a reference diagram for describing a method of aligning a teeth model template to a tooth mass in an intraoral image, according to an embodiment.

The teeth model template 200 may indicate 3D teeth model data indicating the most ideal set of teeth. The teeth model template 200 may be teeth data in which each template tooth has an ideal shape and the template teeth have an ideal array state, wherein each template tooth of the teeth model template 200 is assigned with a tooth number. The teeth model template 200 may include shape data with respect to each template tooth, position data with respect to each tooth, and a tooth number of each tooth. Referring to FIG. 9, for example, the teeth model template 200 may include 14 teeth, and the teeth are sequentially assigned with tooth numbers 1 to 14, starting from a left back tooth.

The data processing device 100 may align the teeth model template 200 to the tooth mass 510. The teeth model template 200 may have position information according to a unique coordinate system of the teeth model template 200, and the tooth mass 510 may also have position information according to a unique coordinate system determined as the intraoral image is obtained by the intraoral scanner. To align the teeth model template 200 to the tooth mass 510 may indicate to position the teeth model template 200 at a corresponding position of the tooth mass 510 by using the position information of the tooth mass 510 and the position information of the teeth model template 200. When the data processing device 100 aligns the teeth model template 200 to the tooth mass 510, various aligning algorithms may be used. For example, a general algorithm, such as ICP, may be used. The ICP is an algorithm for minimizing a space between two point clouds, which is used to reconstruct a 2D or a 3D surface from different scan data. The ICP algorithm fixes a point cloud referred to as a reference and modifies a point cloud referred to as a source to best match the reference. The ICP algorithm may align a 3D model by repeatedly correcting modification (combination of translation and rotation) required to minimize an error metric indicating a distance from the source to the reference. In addition to the ICP, the aligning algorithm may include various algorithms, such as a Kabsch algorithm, etc.

When the data processing device 100 uses the ICP algorithm to align the teeth model template 200 to the tooth mass 510 extracted from the intraoral image 500, a point cloud corresponding to the tooth mass 510 may become the reference, and a point cloud corresponding to the teeth model template 200 may become the source.

After the data processing device 100 may search for a tooth having the closest shape to a first tooth in the tooth mass 510 from the teeth model template 200, the data processing device 100 may determine that a tooth corresponding to the tooth number 1 in the teeth model template 200 may be the tooth having the closest shape to the first tooth in the tooth mass 510. As described above, by aligning the teeth model template 200 to the tooth mass 510 by searching for, from the teeth model template 200, and positioning, the tooth having the closest shape to each tooth of the tooth mass 510, aligning data 900 of the tooth mass and the teeth model template may be obtained. Referring to FIG. 9, the aligning data 900 shows the teeth model template 200 being overlaid and aligned to the tooth mass 510, wherein, in the aligning data 900, the tooth mass 510 is indicated by substantial lines and the teeth model template 200 is indicated by dotted lines.

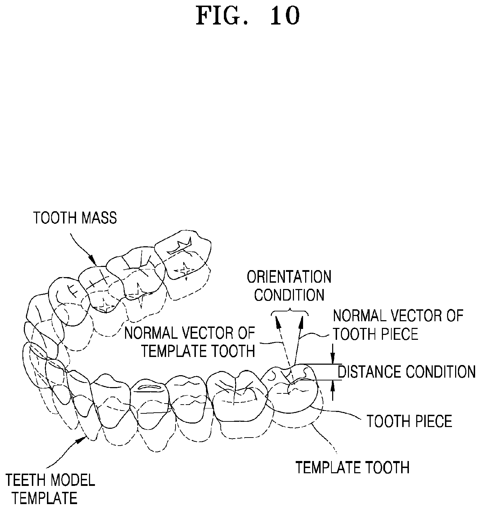

FIG. 10 is a reference diagram for describing a method of obtaining a mapping relationship between each tooth piece of the tooth mass and each template tooth, according to an embodiment.

According to an embodiment, in order to obtain the mapping relationship between each tooth piece of the tooth mass and each template tooth, the data processing device 100 may identify the template tooth corresponding to the tooth piece by using an orientation condition (a normal condition) of the template tooth and the tooth piece. The orientation condition may indicate a condition for identifying the tooth piece corresponding to the template tooth by using a normal vector of the template tooth and a normal vector of the tooth piece. In more detail, the data processing device 100 may identify whether the template tooth and the tooth piece correspond to each other by using an angle of the normal vector at a vertex of each template tooth and the normal vector at a crossing point at which the normal vector meets the tooth piece of the intraoral image. When the angle between the normal vector on the vertex of each template tooth and the normal vector on the crossing point at which the normal vector meets the tooth piece of the intraoral image is less than a threshold angle, the data processing device 100 may identify that the template tooth and the tooth piece correspond to each other, and when the angle is greater than or equal to the threshold angle, the data processing device 100 may identify that the template tooth and the tooth piece do not correspond to each other.

According to an embodiment, in order to obtain the mapping relationship between each tooth piece of the tooth mass and each template tooth, the data processing device 100 may identify the template tooth corresponding to the tooth piece by using a distance condition and an orientation condition of the template tooth and the tooth piece. According to an embodiment, the data processing device 100 may identify, as the tooth piece corresponding to the template tooth, a tooth piece within a predetermined distance from the vertex of each template tooth, the tooth piece satisfying the orientation condition described above.

The data processing device 100 may obtain the mapping relationship between each tooth piece of the tooth mass and all of the template teeth of the teeth model template, by identifying the template teeth corresponding to the tooth piece by using the orientation condition or by using the orientation condition and the distance condition as described with reference to FIG. 10, with respect to all vertexes of the template teeth.

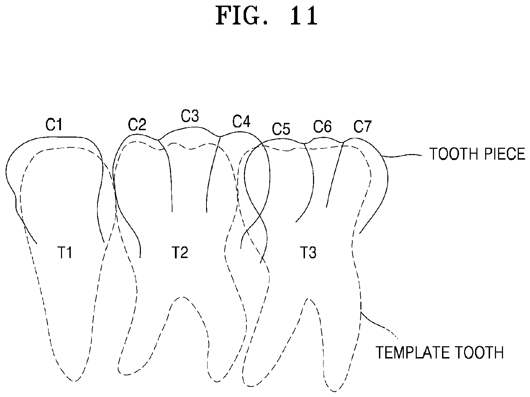

Hereinafter, the method of obtaining the mapping relationship between each tooth piece of the tooth mass and each template tooth by using the orientation condition and the distance condition will be described in detail by referring to FIGS. 11 to 15.

FIG. 11 illustrates a portion of the data in which the tooth mass and the teeth model template are aligned to each other, according to an embodiment.

For the description of the method of obtaining the mapping relationship between the template teeth and the tooth pieces of the tooth mass, it is considered that a portion of the aligning data may include, for example, template teeth T1,T2, and T3 and tooth pieces C1, C2, C3, C4, C5, C6, and C7 as shown in FIG. 11.

The template teeth illustrated in FIG. 11 include, for example, the root of the teeth and the crowns, which are the teeth portions exposed above the gingiva. However, in addition to the shape including the root of the teeth as illustrated in FIG. 11, the template teeth used for the alignment may also include a shape including only the crowns exposed above the gingiva without including the root of the teeth. Referring to FIG. 11, the portion used for the alignment corresponds to the crowns of the template teeth, and thus, the root portions of the template teeth may operate as noise in the aligning operation. Thus, when the template teeth including only the crown portions and not including the root of the teeth is used, the possibility of an aligning error which may be caused by the root of the teeth may be reduced.

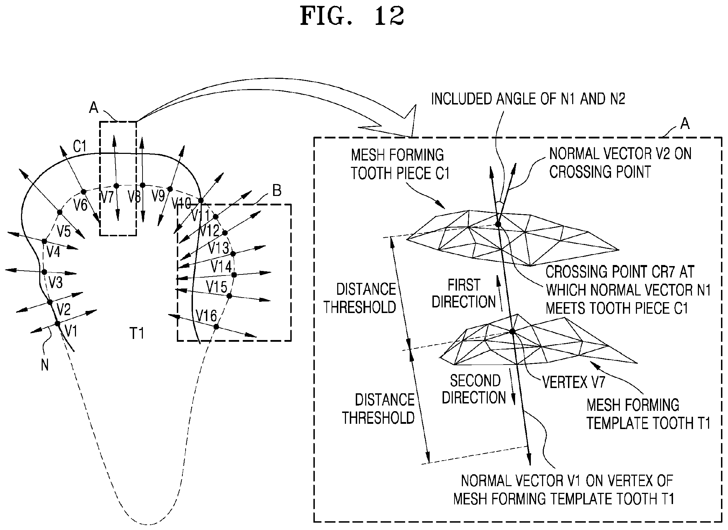

FIG. 12 is a reference diagram for describing a method of obtaining a mapping relationship between each tooth piece of a tooth mass and each template tooth by using an orientation condition and a distance condition, according to an embodiment.

Referring to FIG. 12, the method of obtaining the mapping relationship is described, for example, by using the template tooth T1 and the tooth piece C1.

With respect to all vertexes of a polygonal mesh structure forming the template tooth T1, the data processing device 100 may search for a crossing point at which a normal vector V at each vertex meets a polygonal mesh structure forming a tooth piece. Also, the data processing device 100 may identify an angle formed by the normal vector at the searched crossing point with respect to the mesh structure of the tooth piece and the normal vector on the vertex of the template tooth T1. When the angle formed by the normal vector at a certain vertex of the template tooth and the normal vector at the tooth piece is less than or equal to a threshold value, the mapping relationship indicating thon the vertex of the corresponding template tooth corresponds to the searched tooth piece may be determined. By performing this operation on all of the vertexes of the polygonal mesh structure forming the template tooth T1, the data processing device 100 may identify the tooth piece in the mapping relationship, with respect to all of the vertexes of the template tooth T1. FIG. 12 illustrates some vertexes V1 to V16 from among the vertexes of the polygonal mesh structure forming the template tooth T1.

The method of obtaining the mapping relationship by using the orientation condition and the distance condition will be described in detail, with reference to region A including the vertex V7 forming the template tooth T1.

The data processing device 100 may search for a crossing point CR7 at which a normal vector N1 on the vertex V7 of the polygonal mesh structure forming the template tooth T1 meets a mesh surface forming the tooth piece C1 in a first direction. The crossing point CR7 at which the normal vector N1 meets the mesh surface forming the tooth piece C1 may be a vertex of a mesh structure forming the tooth piece C1 or a surface of a polygon of the mesh structure forming the tooth piece C1.

The data processing device 100 may obtain a normal vector N2 on the crossing point CR7 at which the normal vector V1 meets the mesh surface forming the tooth piece C1. When the crossing point CR7 is on the vertex of the tooth piece C1, the normal vector N2 may be a normal vector on the vertex CR7, and when the crossing point CR7 is on a surface of a triangle of the tooth piece C1, the normal vector N2 may be a normal vector on the surface of the triangle.

The data processing device 100 may identify an included angle formed by the normal vector N1 and the normal vector N2, and when the included angle is less than a threshold value, may determine thon the vertex V7 of the template tooth T1 and the tooth piece C1 are mapped to each other. Thus, the data processing device 100 may obtain the mapping relationship of V7:C1 with respect to the vertex V7 of the template tooth T1. For example, the threshold value of the included angle for determining whether or not mapping is achieved may be determined based on at least one of similarity information of the template tooth and an actually scanned tooth or the reliability (the accuracy) of the aligning algorithm. The threshold value of the included angle for determining whether or not mapping is achieved may be determined to be various values by considering the condition described above, for example, in a range of 20 degrees to 45 degrees. For example, when the threshold value of the included angle for determining whether or not mapping is achieved is determined to be 30 degrees, it may be determined that a template tooth is mapped to a corresponding tooth piece when the included angle is less than 30 degrees. Also, it may be determined that the corresponding template tooth is not mapped to the corresponding tooth piece, when the included angle is greater than or equal to 30 degrees.

According to an embodiment, when the data processing device 100 searches for the crossing point at which the normal vector N on the vertex of the polygonal mesh structure forming the template tooth T1 meets the polygonal mesh structure forming the tooth piece, the data processing device 100 may search for the crossing point not only in the first direction, but also in a second direction. For example, with respect to region B, surfaces of the tooth piece C1 are inside the vertexes V11 to V16 of the mesh structure forming the template tooth T1. Thus, in the case of region B, the data processing device 100 may search for the crossing point of the normal vector on the vertex of the template tooth and the mesh structure forming the tooth piece C1 in the second direction.

According to an embodiment, when obtaining the mapping relationship between the template tooth and the tooth piece, the data processing device 100 may further use a distance condition, in addition to the orientation condition using the normal vector as described above. That is, when the crossing point at which the normal vector on the vertex of the template tooth T1 meets the mesh surface of the tooth piece C1 is within a predetermined distance threshold, the data processing device may determine whether the orientation condition is satisfied, and when the crossing point is not within the predetermined distance threshold, the data processing device may exclude the determining of the mapping relationship, without determining whether or not the orientation condition is satisfied. The distance threshold may be determined based on at least one of similarity information of the template tooth and the actually scanned tooth shape or the reliability (accuracy) of the aligning algorithm. The distance threshold may be determined to be various values by considering the condition described above. For example, when the data processing device 100 determines the distance threshold to be 0.5 mm, the data processing device 100 may search for the crossing point within 0.5 mm from the vertex of the template tooth T1. The data processing device 100 may exclude the crossing point not within 0.5 mm from the vertex of the template tooth T1 from the mapping relationship, even when the corresponding crossing point satisfies the orientation condition. The above example, 0.5 mm, is only an example, and the distance threshold may be variously determined based on an experiential or experimental value. This may indicate that a tooth piece portion is not to be considered to have a mapping relationship, when the tooth piece portion is distanced far away from the vertex of the template tooth T1 even when the tooth piece portion is similar with respect to the orientation.

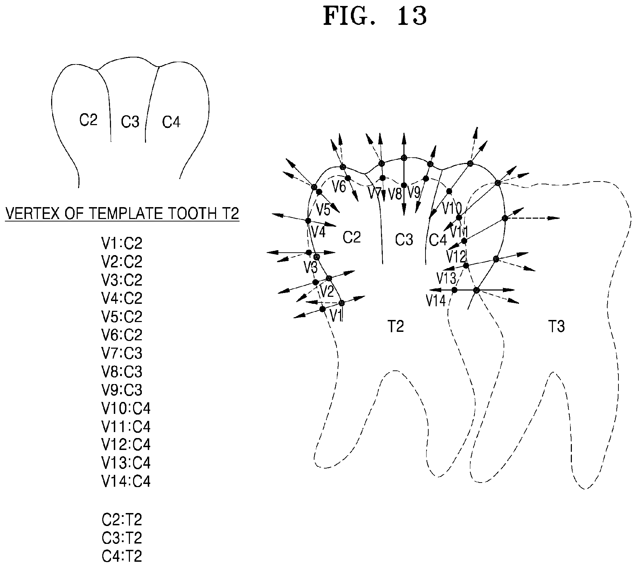

FIG. 13 is a reference diagram for describing a method of obtaining a mapping relationship between each tooth piece of a tooth mass and each template tooth by using an orientation condition and a distance condition, according to an embodiment.

Referring to FIG. 13, a tooth in an intraoral image may be separated into tooth pieces C2, C3, and C4. In this case, a mapping relationship between the template tooth T2 and the tooth pieces C2, C3, and C3 will be described.

Though a mesh surface of the template tooth T2 may include a greater number of vertexes, FIG. 13 illustrates vertexes V1 to V14 of the template tooth T2, for convenience of explanation. The data processing device 100 may summarize the mapping relationship based on an included angle of a normal vector at each vertex of the template tooth T2 and the normal vector at a crossing point at which the normal vector meets a mesh surface of the tooth pieces C2, C3, and C4, thereby obtaining the mapping relationships of V1 :C2, V2:C2, V3:C2, V4:C2, V5:C2, V6:C2, V7:C3, V8:C3, V9:C3, V 10: C4, V11 :C4, V12:C4, V13:C4, and V14:C4.

To summarize the mapping relationship between each vertex of the template tooth T2 and the tooth piece, it may be identified that each of the tooth pieces C2, C3, and C4 is mapped to the template tooth T2.

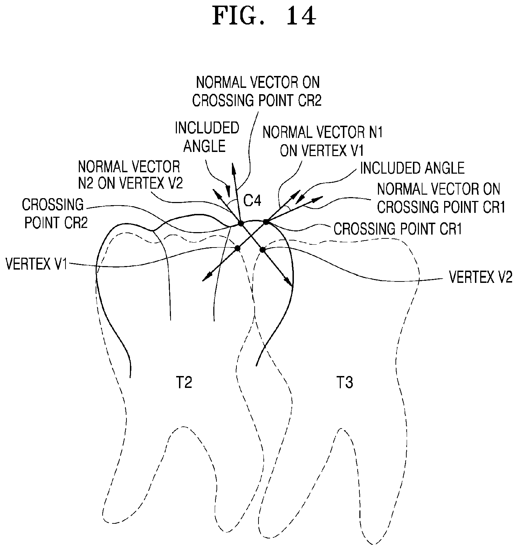

FIG. 14 is a reference diagram for describing a case where one tooth piece is repeatedly mapped to a plurality of template teeth.

A certain tooth piece of an intraoral image may have a mapping relationship in which the tooth piece is mapped to the plurality of template teeth. The data processing device 100 may exclude the tooth piece repeatedly mapped to the plurality of tooth pieces from the mapping relationship.

Referring to FIG. 14, the tooth piece C4 is mapped to the template tooth T2 and also to the template tooth T3. That is, when an included angle formed by a normal vector on the vertex V1 of the template tooth T2 and the normal vector on the crossing point CR1 at which the normal vector meets the tooth piece C4 is less than a threshold value, the data processing device 100 may determine that the template tooth T2 is mapped to the tooth piece C4. Also, when an included angle formed by the normal vector on the vertex V2 of the template tooth T3 and the normal vector on the crossing point CR2 at which the normal vector meets the tooth piece C4 is less than the threshold value, the data processing device 100 may determine that the template tooth T3 is mapped to the tooth piece C4.

As described above, when the tooth piece C4 is mapped to both of the template tooth T2 and the template tooth T3, that is, when the tooth piece C4 is repeatedly mapped to the template tooth T2 and the template tooth T3, the data processing device 100 may exclude the tooth piece C4 repeatedly mapped to the plurality of template teeth from the mapping relationship.

Selectively, the data processing device 100 may not exclude the tooth piece repeatedly mapped to the plurality of template teeth and may map the tooth piece to the template tooth determined to be more similar to the tooth piece than other template teeth.

The similarity may be assessed by appropriately combining the mapping frequency, the region, the angle, the distance, etc.

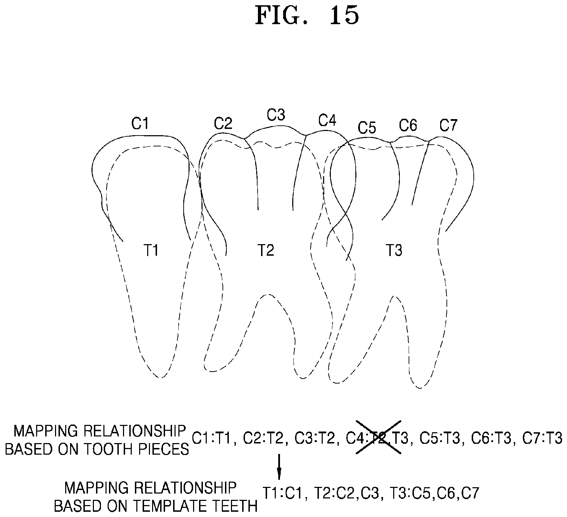

FIG. 15 is a reference diagram for describing a method of individualizing teeth of an intraoral image by collecting one or more tooth pieces mapped to template teeth by using a mapping relationship between the tooth pieces and the template teeth, according to an embodiment.

Referring to FIG. 15, the data processing device 100 may obtain the mapping relationships of C1:T1, C2:T2, C3:T2, C4:T2, T3, C5:T3, C6:T3, and C7:T3 with respect to tooth pieces C1,C2, C3, C4, C5, C6, and C7 of the intraoral image, by using the orientation condition and the distance condition as described above. The data processing device 100 may reversely take the mapping relationships and may collect, based on the template teeth, one or more tooth pieces mapped to each template tooth, in order to individualize the tooth corresponding to each template tooth. That is, the data processing device 100 may obtain the corresponding relationships of T1:C1, T2:C2, C3, T3:T5, C6, and C7, by summarizing the mapping relationships described above based on the template teeth. This may indicate that the template tooth T1 corresponds to the tooth piece C1, the template tooth T2 corresponds to the tooth pieces C2 and C3, and the template tooth T3 corresponds to the tooth pieces C5, C6, and C7. In the case of the tooth piece C4, the tooth piece C4 is repeatedly mapped to the plurality of different template teeth T2 and T3, and thus, the tooth piece C4 may not be used and may be excluded from the operation of collecting one or more tooth pieces mapped to each template tooth. As described above, an empty space which may occur when the teeth are individualized due to the tooth piece excluded from the collection of the tooth pieces may be naturally compensated for by surface reconstruction, mesh hole filling, mesh reconstruction, etc.

As described above, the data processing device 100 may determine the tooth corresponding to each template tooth by collecting one or more tooth pieces corresponding to each template tooth. The data processing device 100 may complete a teeth model through appropriate post processing, after determining the tooth corresponding to each template tooth. After the data processing device 100 determines the tooth corresponding to each template tooth, the data processing device 100 may determine, by using information about the template tooth, information of the corresponding tooth, that is, information related to a position, a posture, and pose (or an attitude) of the tooth, a tooth number of the tooth, or the like. In detail, the data processing device 100 may transfer posture information of the template tooth, for example, buccal, lingual, distal, mesial information, etc., to the corresponding scanned tooth.

The intraoral image processing method according to an embodiment of the disclosure may be realized as a program command which may be executed by various computer devices and may be recorded on a computer-readable recording medium. Also, according to an embodiment of the disclosure, a computer-readable storage medium having recorded thereon one or more programs including one or more instructions for executing the intraoral image processing method may be provided.

The computer-readable medium may include a program command, a data file, a data structure, etc. individually or in a combined fashion. Here, examples of the computer-readable storage medium include magnetic media, such as hard discs, floppy discs, and magnetic tapes, optical media, such as compact disc-read only memories (CD-ROMs) and digital versatile discs (DVDs), magneto-optical media, such as floptical discs, and hardware devices configured to store and execute program commands, such as ROMs, RAMs, and flash memories.

Here, a machine-readable storage medium may be provided in a form of a non-transitory storage medium. Here, the "non-transitory storage medium" may denote a tangible storage medium. Also, the "non-transitory storage medium" may include a buffer in which data is temporarily stored.

According to an embodiment, the intraoral image processing method according to various embodiment of the disclosure may be provided by being included in a computer program product. The computer program project may be distributed in the form of a device-readable storage medium (for example, CD-ROM). Alternatively, the computer program may be directly or through online distributed (e.g. download or upload) between two user devices (e.g., smartphones) through an application store (e.g. Play Store, etc.). In detail, the computer program product according to an embodiment may include a storage medium having recorded thereon a program including at least one instruction for executing the intraoral image processing method according to an embodiment.

Although embodiments of the disclosure are described in detail above, the scope of the claims of the disclosure is not limited thereto, and various modifications and alterations by one of ordinary skill in the art using basic concept of the disclosure defined by the following claims are also included in the scope of the claims of the disclosure.

## Claims

1. An intraoral image processing method comprising:
obtaining an intraoral image generated by scanning teeth;
aligning a teeth model template including a plurality of template teeth to teeth included in the intraoral image;
obtaining a plurality of tooth pieces by separating the teeth of the intraoral image; and
individualizing the teeth of the intraoral image by collecting one or more tooth pieces corresponding to each of the aligned template teeth.

2. The intraoral image processing method of claim 1, wherein the individualizing of the teeth of the intraoral image comprises:
obtaining a mapping relationship between each tooth piece of the intraoral image and each of the template teeth by identifying the tooth piece corresponding to each aligned template tooth; and
collecting the one or more tooth pieces mapped to each template tooth by using the mapping relationship.

3. The intraoral image processing method of claim 1, wherein the aligning of the teeth model template including the plurality of template teeth to the teeth included in the intraoral image comprises positioning the plurality of template teeth of the teeth model template to correspond to the teeth included in the intraoral image.

4. The intraoral image processing method of claim 1, wherein the obtaining of the plurality of tooth pieces by separating the teeth of the intraoral image comprises separating each tooth into one or more tooth pieces by separating the teeth according to a curvature distribution.

5. The intraoral image processing method of claim 1, wherein the individualizing of the teeth of the intraoral image comprises identifying each tooth piece corresponding to each template tooth by using an orientation condition of each template tooth and each tooth piece.

6. The intraoral image processing method of claim 5, wherein the identifying of each tooth piece corresponding to each template tooth by using the orientation condition comprises:
identifying a crossing point at which a normal vector on each vertex of a three-dimensional (3D) mesh forming the template tooth intersects a 3D mesh forming the tooth piece; and
based on an angle between a normal vector on the identified crossing point with respect to the tooth piece and the normal vector on the vertex being less than a threshold value, determining that the tooth piece corresponds to the template tooth.

7. The intraoral image processing method of claim 1, wherein the individualizing of the teeth of the intraoral image comprises identifying each tooth piece corresponding to each template tooth by using a distance condition of each template tooth and each tooth piece, and
the identifying of each tooth piece corresponding to each template tooth comprises:
identifying a crossing point at which a normal vector on each vertex of a 3D mesh forming the template tooth intersects a 3D mesh forming the tooth piece; and
based on a distance from the vertex to the crossing point being less than a threshold value, determining that the tooth piece corresponds to the template tooth.

8. The intraoral image processing method of claim 1, wherein the individualizing of the teeth of the intraoral image comprises identifying each tooth piece corresponding to each template tooth by using a distance condition and an orientation condition of each template tooth and each tooth piece, and
the identifying of each tooth piece corresponding to each template tooth by using the distance condition and the orientation condition comprises:
identifying, as a crossing point at which a normal vector on each vertex of a 3D mesh forming the template tooth intersects a 3D mesh forming the tooth piece, the crossing point within a predetermined distance from the vertex; and
based on an angle between a normal vector on the identified crossing point with respect to the tooth piece and the normal vector on the vertex being less than a threshold value, determining that the tooth piece corresponds to the template tooth.

9. An intraoral image processing device comprising a processor and a memory,
wherein the processor is configured to execute one or more instructions stored in the memory to:
obtain an intraoral image generated by scanning teeth;
align a teeth model template including a plurality of template teeth to teeth included in the intraoral image;
obtain a plurality of tooth pieces by separating the teeth of the intraoral image; and
individualize the teeth of the intraoral image by collecting one or more tooth pieces corresponding to each of the aligned template teeth.

10. The intraoral image processing device of claim 9, wherein, in order to individualize the teeth of the intraoral image, the processor is further configured to execute the one or more instructions stored in the memory to:
obtain a mapping relationship between each tooth piece of the intraoral image and each of the template teeth by identifying the tooth piece corresponding to each aligned template tooth; and
collect the one or more tooth pieces mapped to each template tooth by using the mapping relationship.

11. The intraoral image processing device of claim 9, wherein, in order to align the teeth model template including the plurality of template teeth to the teeth included in the intraoral image, the processor is further configured to execute the one or more instructions stored in the memory to position the plurality of template teeth of the teeth model template to correspond to the teeth included in the intraoral image.

12. The intraoral image processing device of claim 9, wherein, in order to obtain the plurality of tooth pieces by separating the teeth of the intraoral image, the processor is further configured to execute the one or more instructions stored in the memory to separate each tooth into one or more tooth pieces by separating the teeth according to a curvature distribution.

13. The intraoral image processing device of claim 9, wherein, in order to individualize the teeth of the intraoral image, the processor is further configured to execute the one or more instructions stored in the memory to identify each tooth piece corresponding to each template tooth by using an orientation condition of each template tooth and each tooth piece.

14. The intraoral image processing device of claim 13, wherein, in order to identify each tooth piece corresponding to each template tooth by using the orientation condition, the processor is further configured to execute the one or more instructions stored in the memory to:
identify a crossing point at a normal vector on each vertex of a three-dimensional (3D) mesh forming the template tooth intersects a 3D mesh forming the tooth piece; and
based on an angle between a normal vector on the identified crossing point with respect to the tooth piece and the normal vector on the vertex being less than a threshold value, determine that the tooth piece corresponds to the template tooth.

15. The intraoral image processing device of claim 9, wherein, in order to individualize the teeth of the intraoral image, the processor is further configured to execute the one or more instructions stored in the memory to:
identify each tooth piece corresponding to each template tooth by using a distance condition of each template tooth and each tooth piece, and
in order to identify each tooth piece by using the distance information,
identify a crossing point at which a normal vector on each vertex of a 3D mesh forming the template tooth intersects a 3D mesh forming the tooth piece; and
based on a distance from the vertex to the crossing point being less than a threshold value, determine that the tooth piece corresponds to the template tooth.

16. The intraoral image processing device of claim 9, wherein, in order to individualize the teeth of the intraoral image, the processor is further configured to execute the one or more instructions stored in the memory to:
identify each tooth piece corresponding to each template tooth by using a distance condition and an orientation condition of each template tooth and each tooth piece;
in order to identify each tooth piece by using the distance information and the orientation condition,
identify, as a crossing point at which a normal vector on each vertex of a 3D mesh forming the template tooth intersects a 3D mesh forming the tooth piece, the crossing point within a predetermined distance from the vertex; and
based on an angle between a normal vector on the identified crossing point with respect to the tooth piece and the normal vector on the vertex being less than a threshold value, determine that the tooth piece corresponds to the template tooth.

17. A computer-readable recording medium having recorded thereon a program comprising one or more instructions for executing, on a computer, an intraoral image processing method comprising:
obtaining an intraoral image generated by scanning teeth;
aligning a teeth model template including a plurality of template teeth to teeth included in the intraoral image;
obtaining a plurality of tooth pieces by separating the teeth of the intraoral image; and
individualizing the teeth of the intraoral image by collecting one or more tooth pieces corresponding to each of the aligned template teeth.
